# EUROPEAN PATENT APPLICATION

(11) **EP 4 769 004 A1**
(43) Date of publication of application: **01.07.2026**
(21) Application number: 24223493.8
(22) Date of filing: 27.12.2024
(51) Int. Cl.: G02B 27/00, G02B 23/24, G03B 37/00

(54) **IMAGING ELEMENT CLEANING DEVICE AND METHOD OF OPERATING THE SAME**

(71) Applicant: Industrial Technology Research Institute, Hsinchu 31040 Taiwan (TW)
(72) Inventor: HSU, Chih-Cheng, Zhubei City (TW); LIN, Chun-Chuan, Hsinchu City (TW); LU, Shih-Chieh, Hsinchu City (TW); WU, Kun-Ta, Zhudong Township (TW)
(74) Representative: Ström & Gulliksson AB

(57) **Abstract**

An imaging element cleaning device for cleaning a lens of an imaging element is provided, including a cleaning element and a driving component. The cleaning element includes a flexible sleeve that is hollow and includes a distal end and a proximal end opposite to the distal end, a cleaning part partially covering the distal end of the flexible sleeve, and a slit part penetrating through the cleaning part. The driving component includes a tube, which includes a distal end and a proximal end opposite to the distal end for the imaging element to pass through, and the flexible sleeve of the cleaning element is sleeved on the distal end of the tube, wherein when the driving component is driven to move the cleaning element, the lens of the imaging element passes through the slit part to be cleaned by the cleaning part.

## Description

### BACKGROUND

### Field of Invention

The technical field relates to imaging element cleaning device and method of operating the same.

### Description of Related Art

Endoscopes have been used in clinical practice for decades. Endoscopes are medical instruments that enter the body through tubes to observe the internal conditions of the body. Endoscopes are widely used in the examination and treatment of various body cavities, such as digestive tract, respiratory tract, and urinary tract. Because of the complex working environment, endoscope lens is easily contaminated by pollutants such as body fluids, blood, mucus and tissue fragments. Conventional anti-adhesion methods for endoscope lens include tissue expanders expanding the tissues in the subject's body to prevent tissue fluid from adhering to the endoscope lens surface.

### SUMMARY

One embodiment of the present disclosure provides an imaging element cleaning device for cleaning a lens of an imaging element, the imaging element cleaning device comprises a cleaning element and a driving component. cleaning element comprises a flexible sleeve, a cleaning part, and a slit part. The flexible sleeve is hollow, and comprises a distal end and a proximal end opposite to the distal end. The cleaning part partially covers the distal end of the flexible sleeve. The slit part penetrates through the cleaning part. The driving component comprises a tube comprising a distal end and a proximal end opposite to the distal end for the imaging element to pass through, and the flexible sleeve of the cleaning element is sleeved on the distal end of the tube, wherein when the driving component is driven to move the cleaning element, the lens of the imaging element passes through the slit part for cleaning by the cleaning part.

Another embodiment of the present disclosure provides a method of operating imaging element cleaning device, comprising providing the imaging element cleaning device as above mentioned and an imaging element passing through the tube of the driving component and the slit part of the cleaning element, wherein when a lens of the imaging element is contaminated, the driving component is driven to move the cleaning element, and the lens of the imaging element is moved toward a proximal end of the tube and passes through the slit part for cleaning by the cleaning part.

### BRIEF DESCRIPTION OF THE DRAWINGS

Aspects of the present disclosure are best understood from the following detailed description when read with the accompanying figures. It is noted that, in accordance with the standard practice in the industry, various features are not drawn to scale. In fact, the dimensions of the various features may be arbitrarily increased or reduced for clarity of discussion. The disclosure can be more fully understood by reading the following detailed description of the embodiment, with reference made to the accompanying drawings as follows:
Fig. 1 depicts a perspective view of an imaging element cleaning device according to an embodiment of the present disclosure.
Figs. 2A to 2E depict top views and corresponding cross-sectional views of cleaning elements according to some embodiments of the present disclosure.
Figs. 3A to 3D depict top views of cleaning elements according to some embodiments of the present disclosure.
Fig. 4 depicts a perspective view showing the use of a guiding element of the imaging element cleaning device according to another embodiment of the present disclosure.
Fig. 5 depicts a perspective view of the imaging element cleaning device with an automatic control driving device according to an embodiment of the present disclosure.
Fig. 6 depicts a side view and a partial cross-sectional view of Fig. 5.
Fig. 7 depicts a partial cross-sectional view of Fig. 5.
Fig. 8 depicts a perspective view showing the use of the guiding element of the imaging element cleaning device according to an embodiment of the present disclosure.
Fig. 9 depicts a side view showing the use of the automatic control driving device according to another embodiment of the present disclosure.

### DETAILED DESCRIPTION

Further, spatially relative terms, such as "beneath," "over" and the like, may be used herein for ease of description to describe one element or feature's relationship to another element(s) or feature(s) as illustrated in the figures. The spatially relative terms are intended to encompass different orientations of the device in use or operation in addition to the orientation depicted in the figures. The apparatus may be otherwise oriented (rotated 90 degrees or at other orientations) and the spatially relative descriptors used herein may likewise be interpreted accordingly.

The terminology used herein is for the purpose of describing particular embodiments only and is not intended to be limiting of the present disclosure. As used herein, the singular forms "a", "an" and "the" are intended to include the plural forms as well, unless the context clearly indicates otherwise. It will be further understood that the terms "comprises" and/or "comprising", or "includes" and/or "including" or "has" and/or "having" when used in this specification, specify the presence of stated features, regions, integers, steps, operations, elements, and/or components, but do not preclude the presence or addition of one or more other features, regions, integers, steps, operations, elements, components, and/or groups thereof.

Further, when a number or a range of numbers is described with "about," "approximate," and the like, the term is intended to encompass numbers that are within a reasonable range considering variations that inherently arise during manufacturing as understood by one of ordinary skill in the art. For example, the number or range of numbers encompasses a reasonable range including the number described, such as within +/-10% of the number described, based on known manufacturing tolerances associated with manufacturing a feature having a characteristic associated with the number. Still further, the present disclosure may repeat reference numerals and/or letters in the various examples. This repetition is for the purpose of simplicity and clarity and does not in itself dictate a relationship between the various embodiments and/or configurations discussed.

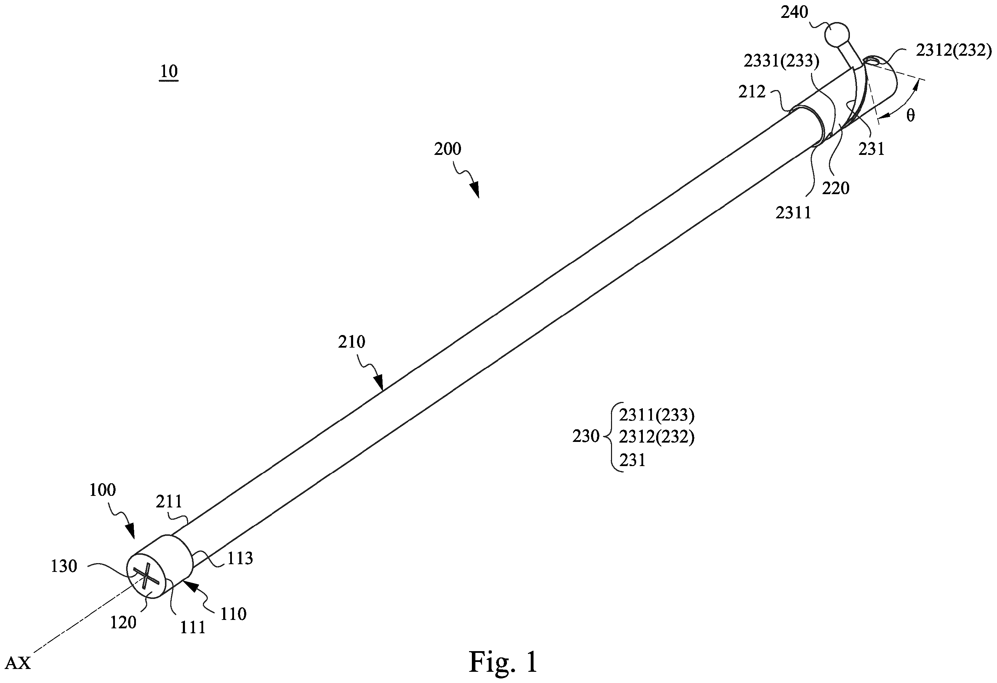

Please refer to Fig. 1, Fig. 1 depicts a perspective view of an imaging element cleaning device according to an embodiment of the present disclosure. An imaging element cleaning device 10 includes a cleaning element 100 and a driving component 200. The cleaning element 100 includes a flexible sleeve 110, a cleaning part 120, and a slit part 130. In some examples, a material of the cleaning element 100 is biocompatible material including, but not limited to silicone, rubber, resin, or a combination thereof; the resin includes, but is not limited to acrylonitrile, butadiene, acrylonitrile-butadiene-styrene (ABS). In some examples, the cleaning element 100 is integrally formed. The flexible sleeve 110 is hollow, the flexible sleeve 110 includes a distal end 111, an edge 112 located at the distal end 111, and a proximal end 113 opposite to the distal end 111. In some examples, the flexible sleeve 110 is cylindrical and has an axis AX.

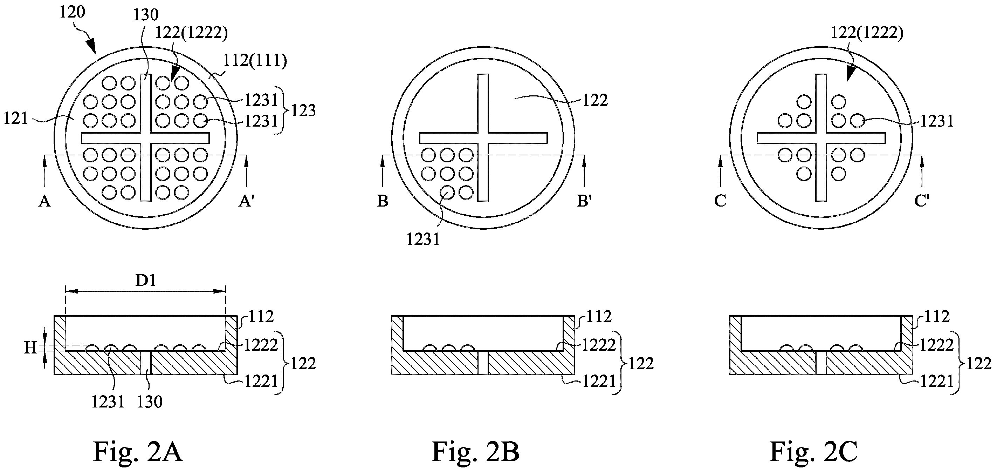

Please refer to Fig. 1 and Fig. 2A at the same time, Fig. 2A depicts a top view and a corresponding cross-sectional view of the cleaning element according to an embodiment of the present disclosure. The cleaning part 120 partially covers the distal end 111 of the flexible sleeve 110. In some examples, cleaning part 120 includes an edge 121, at least one slice 122, and at least one protrusion 123. The edge 121 is located at the distal end of the cleaning part 120, and is adjacent to the edge 112 of the distal end 111 of the flexible sleeve 110. The at least one slice 122 extends from the edge 112 of the distal end 111 of the flexible sleeve 110 toward the axis AX of the flexible sleeve 110 (i.e., extends from the edge 121 of the cleaning part 120 toward the axis AX), in which the at least one slice 122 has an outer surface 1221 and an inner surface 1222 opposite to the outer surface 1221. The at least one protrusion 123 is disposed on the inner surface 1222 of the at least one slice 122, and is used as contacting and cleaning the lens of the imaging element. In some examples, flexible sleeve 110 has an inner diameter D1, including but not limited to about 4 mm to about 7 mm, such as 4 mm, 4.5 mm, 5 mm, 5.5 mm, 6 mm, 6.5 mm, 7 mm, or any value between any two of these values. It is worth noting that the flexible sleeve 110 has the inner diameter D1 that can be slightly larger than an outer diameter of the lens of the cleaning imaging element, and therefore is not limited to the aforementioned numerical range.

In some examples, the cleaning part 120 includes, but is not limited to at least two slices 122, at least three slices 122, at least four slices 122, at least five slices 122, or at least six slices 122. The more the slices 122 there are, the less the resistance to the imaging element (such as an endoscope) passing through the slice 122. Therefore, a number of the slices 122 can be adjusted according to needs. In some examples, the cleaning part 120 includes the at least four slices 122 extend from the edge 112 of the distal end 111 of the flexible sleeve 110 toward the axis AX of the flexible sleeve 110, in which each one of the at least four slices 122 has an outer surface 1221 and an inner surface 1222 opposite to the outer surface 1221. The at least one protrusion 123 is disposed on the inner surface 1222 of one of the at least four slices 122. In some examples, a number of the at least one protrusion 123 is at least four protrusions 123 respectively disposed on the inner surfaces 1222 of the at least four slices 122. In some examples, each one of the at least four protrusions 123 includes a plurality of bumps 1231 to improve cleaning efficiency and reduce the number of forward and backward movements and rotations of the cleaning part 120. In some examples, a distribution pattern of the plurality of bumps 1231 is evenly distributed on the inner surface 1222 of each slice 122. In some examples, the bump 1231 has a height H (upper Fig. 2A and a cross-sectional view at lower Fig. 2A from a section line A-A' at upper Fig. 2A) is 0.1 mm to 0.5 mm, such as 0.1 mm, 0.15 mm, 0.2 mm, 0.25 mm, 0.3 mm, 0.35 mm, 0.4 mm, 0.45 mm, 0.5 mm, or any value between any two of these values. In some examples, a percentage of an area of the plurality of bumps 1231 in one of the slices 122 can be adjusted according to the needs, such as about 20% to 90%, includes, but is not limited to 20%, 30%, 40%, 50%, 60%, 70%, 80%, or any value between any two of these values. In some examples, the plurality of bumps 1231 are disposed on one of (such as upper Fig. 2B and a cross-sectional view at lower Fig. 2B from a section line B-B' at upper Fig. 2B), two of, three of, or all of the four slices 122.

Please refer to Fig. 1 and Fig. 2C at the same time, Fig. 2C depicts a top view and a corresponding cross-sectional view of the cleaning element according to another embodiment of the present disclosure. The difference from Fig. 2A is that the distribution pattern of each one of the plurality of bumps 1231 (upper Fig. 2C and a cross-sectional view at lower Fig. 2C from a section line C-C' at upper Fig. 2C) is concentratedly distributed on the inner surface 1222 of each slice 122 adjacent to the axis AX of the flexible sleeve 110 to enhance cleaning a central part of the imaging element lens.

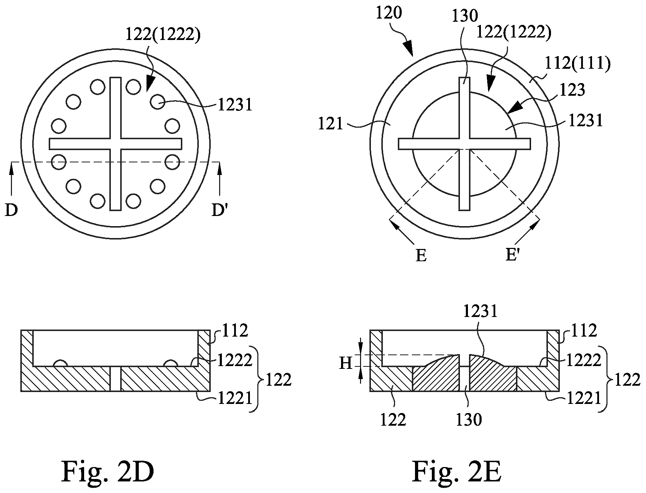

Please refer to Fig. 1 and Fig. 2D at the same time, Fig. 2D depicts a top view and a corresponding cross-sectional view of the cleaning element according to another embodiment of the present disclosure. The difference from Fig. 2A is that the distribution pattern of each one of the plurality of bumps 1231 (upper Fig. 2D and a cross-sectional view at lower Fig. 2D from a section line D-D' at upper Fig. 2D) is concentratedly distributed on the inner surface 1222 of each slice 122 and away from the axis AX of the flexible sleeve 110 to enhance cleaning the lens area of the imaging element lens area.

Please refer to Fig. 1 and Fig. 2E at the same time, Fig. 2E depicts a top view and a corresponding cross-sectional view of the cleaning element according to another embodiment of the present disclosure. Each one of the at least four protrusions 123 includes bump 1231, bump 1231 has a height H increasing from the inner surface 1222 of each of the at least four slices 122 adjacent to the edge 121 of the cleaning part 120 toward a direction of the axis AX, to remove high viscosity liquids. Specifically, the four bumps 1231 are respectively located at the four protrusions 123. When the four bumps 1231 are observed simultaneously, they are thicker near the axis AX and thinner away from the axis AX, that is, the center is thick and the periphery is thin. In some examples, the height H of the bump 1231(upper Fig. 2E and a cross-sectional view at lower Fig. 2E from a section line E-E' at upper Fig. 2E) is gradually increase from 0 mm to 0.2-0.5 mm. In some other examples, the bump 1231 has the height H that increases gradually from the inner surface 1222 of each of the at least four slices 122 adjacent to the edge 112 toward the slit part 130 located at the axis AX. That is, when the inner surface 1222 is a circular plane, a center point of the inner surface 1222 is taken as a center of the circle. The circle drawn from a half distance from the center of the circle to the edge 121 as the radius is the circumference. The bump 1231 has the height H that increases gradually from the circumference to the center. In some examples, the area occupied by the bump 1231 in the slice 122 can be adjusted according to needs, such as about 20% to 80%, including, but not limited to 20%, 30%, 40%, 50%, 60%, 70%, 80%, or any value between any two of these values. In some examples, a material of the protrusion 123 is biocompatible material including, but not limited to silicone, rubber, or a combination thereof. In some examples, the material of the protrusion 123 may be the same as or different from the material of the slice 122.

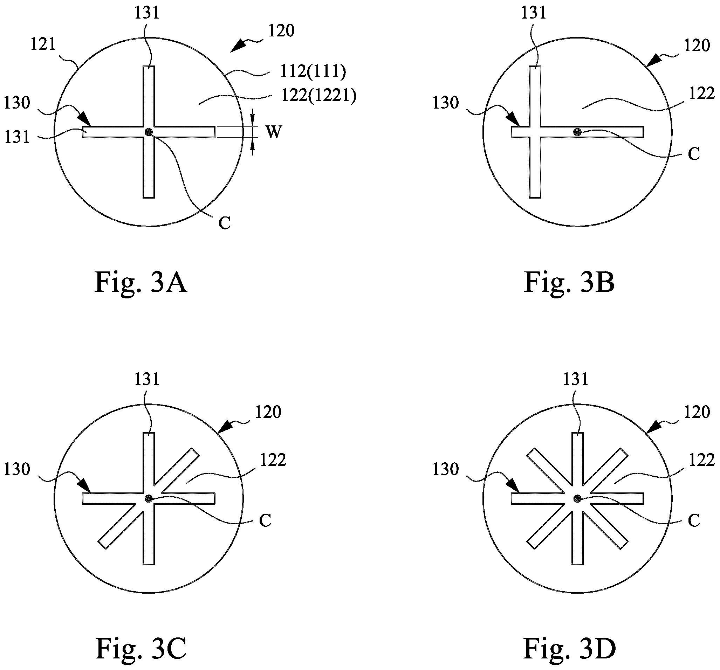

Please refer to Fig. 1, Fig. 2A, and Fig. 3A at the same time, Fig. 3A depicts a top view of the cleaning element according to an embodiment of the present disclosure. The slit part 130 penetrates through the cleaning part 120. In some examples, the slit part 130 includes at least one slit 131, if the at least one slit 131 is a slit 131 penetrating through the edge 112 adjacent to the flexible sleeve 110, a slice 122 is formed (figure not shown); if the at least one slit 131 is a slit 131 penetrating through the edge 112 away from the flexible sleeve 110, two slices 122 are formed. In some examples, the slit part 130 includes at least two slits 131 intersecting each other, and separating into at least three slices 122; four slices 122 are in this example. Specifically, in the top view, two slits 131 intersect each other symmetrically, that is, two slits 131 are symmetrically cross-shaped hollows and intersect at a geometric center C of the cleaning part 120 to form four slices 122 with the same size, to remove the contaminants or tissue fluid contaminated on the lens along the symmetrical slit 131. In some examples, a width W of the slit 131 includes, but is not limited to about 0.2 mm to about 0.4 mm, such as 0.2 mm, 0.25 mm, 0.3 mm, 0.35 mm, 0.4 mm, or any value between any two of these values. It is worth noting that the width W of the slit 131 is not limited to the aforementioned numerical range as long as it can allow the contaminants or tissue fluid to be discharged.

Please refer to Fig. 3B, Fig. 3B depicts a top view of the cleaning element according to an embodiment of the present disclosure. The difference from Fig. 3A is that the slit part 130 includes at least two slits 131 intersecting each other asymmetrically to remove large contaminants and the center of the imaging element lens. Specifically, two slits 131 intersect each other, one of the slits 131 laterally penetrates through the geometric center C of the cleaning part 120, the other one of the slits 131 vertically penetrates through the left side of the cleaning part 120, so that an intersection of the two slits 131 is eccentrically moved to the left to form four slices 122 with different sizes, that is, two large slices 122 and two small slices 122.

Please refer to Fig. 3C, Fig. 3C depicts a top view of the cleaning element according to an embodiment of the present disclosure. The difference from Fig. 3A is that the slit part 130 includes three slits 131 intersecting each other to increase the smoothness of the imaging element entering and exiting the slit part 130. Specifically, two of the three slits 131 intersect each other, a third slit 131 is disposed between the two of the three slits 131, and the intersection of the three slits 131 is located at the geometric center C of the cleaning part 120 to form 6 slices 122, in which two identical large slices 122 and four identical small slices 122. In some examples, the intersection of the three slits 131 is located at the geometric center C of the cleaning part 120, and the three slits 131 intersect each other at the same angle to form six slices 122 with the same size (figure not shown).

Please refer to Fig. 3D, Fig. 3D depicts a top view of the cleaning element according to an embodiment of the present disclosure. The difference from Fig. 3A is that the slit part 130 includes four slits 131 intersecting each other symmetrically to increase the smoothness of the imaging element entering and exiting the slit part 130. Specifically, two alternate slits 131 among the four slits 131 intersect each other perpendicularly, and the intersection of the four slits 131 is located at the geometric center C of the cleaning part 120 to form eight slices 122. The intersection of the four slits 131 is located at the geometric center C of the cleaning part 120, and the intersection angles are the same to form eight slices 122 with the same size. Fig. 3D is only an example, in another examples, the intersection of the four slits 131 is located at the geometric center C of the cleaning part 120, and the intersection angles are different from each other to form eight slices 122 with different sizes.

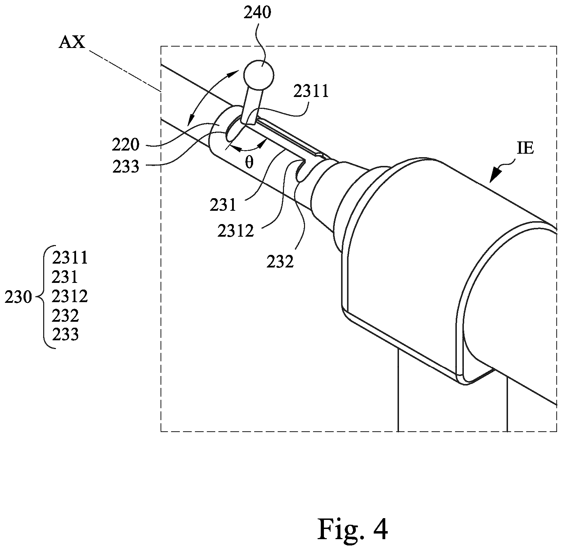

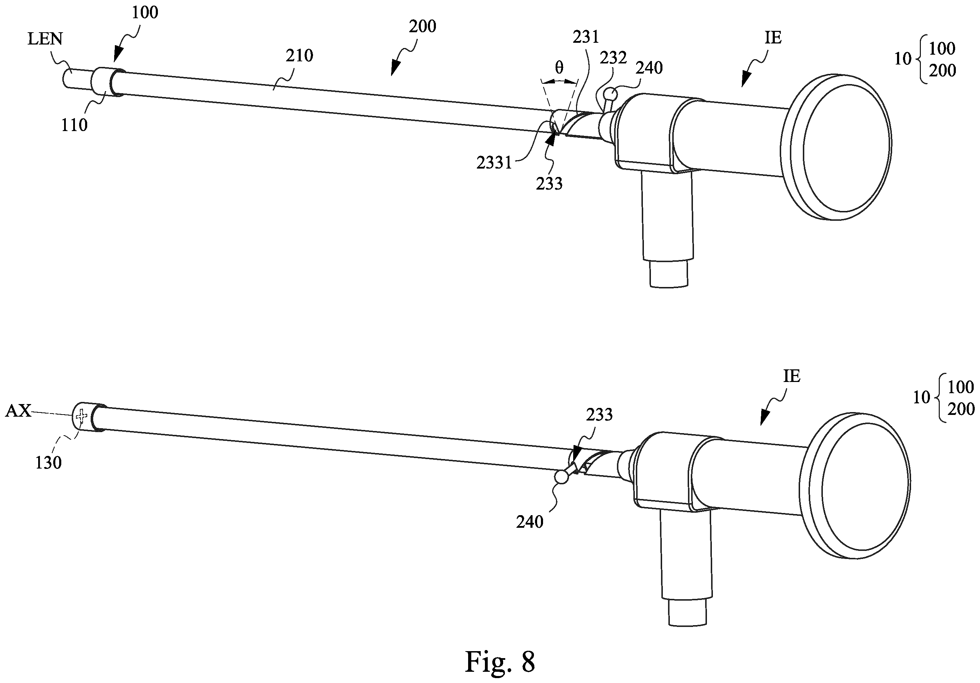

Please refer back to Fig. 1, driving component 200 includes a tube 210, a guiding element 220, a guiding groove 230, and a control handle 240. The tube 210 is used for the imaging element to pass through, the flexible sleeve 110 of the cleaning element 100 is sleeved at the distal end 211 of the tube 210, in which when the driving component 200 is driven to move the cleaning element 100, the lens of the imaging element passes through the slit part 130 for cleaning by the cleaning part 120. In some examples, a material of the tube 210 is biocompatible including, but not limited to stainless steel, such as nickel-free stainless steel to avoid skin irritation. In some examples, the nickel-free stainless steel includes, but is not limited to SS420 stainless steel. The guiding element 220 is sleeved at the proximal end 212 of the tube 210, the guiding groove 230 penetrates through the guiding element 220. In some examples, when the imaging element IE (such as endoscope, as shown in Fig. 4) passes through the tube 210 of the driving component 200, the proximal end of the endoscope guiding element 220 is also disposed at an end of the imaging element IE. In some examples, the shape of the guiding groove 230 can be designed based on the cleaning action that the cleaning element 100 is intended to provide relative to the imaging element IE (for example, the cleaning element 100 is driven to slide relative to the imaging element IE and then is rotated *in situ* by 1/2 turn to clean relative to the lens LEN of the imaging element IE (as shown in Fig. 8 ), or the cleaning element is driven to rotate relative to the imaging element IE and slide to the lens LEN of imaging element IE and then rotate *in situ* by 1/4 turn to clean); in other examples, the shape of the guiding groove 230 allows the control handle 240 to pass through and slides to drive the cleaning element 100 to move, and is not limited to that disclosed in the drawings of the present disclosure.

In some examples as shown in Fig. 1, the guiding groove 230 is spiral, that is, the guiding groove 230 surrounds the tube 210 along a direction of the axis AX. When the imaging element passes through the tube 210 of the driving component 200 and the control handle 240 slides along the guiding groove 230, the tube 210 and the cleaning part 120 will be driven to rotate to clean the lens of the imaging element. In some examples, the guiding groove 230 includes a main groove 231, a first locking slot 232, and a second locking slot 233. Specifically, the main groove 231 is spiral as above mentioned. The first locking slot 232 is disposed at a proximal end 2312 of the main groove 231, when not cleaning (the imaging element is in use), fix a position of control handle 240, and also fix the cleaning element 100. The second locking slot 233 is disposed at a distal end 2311 of the main groove 231, and the second locking slot 233 includes a bottom portion 2331 away from the distal end 2311 of the main groove 231, when cleaning (the imaging element is not in use), an area where the control handle 240 can be moved is limited; such as, after the control handle 240 slides along the main groove 231 to the second locking slot 233 and slides back and forth in the second locking slot 233, at this time, the cleaning element 100 is also rotated *in situ* to clean the lens LEN of the imaging element (as shown in FIG. 8 ); or the control handle 240 may be temporarily stopped during cleaning to fix the position of the control handle 240, for example, the control handle 240 is fixed at the bottom portion 2331 of the second locking slot 233. In some examples, the intersection angle θ of the first locking slot 232 and the main groove 231 and the intersection angle θ of the second locking slot 233 and the main groove 231 are acute angles (as shown in upper Fig. 8) including, but not limited to about 10 degrees to less than 90 degrees, such as 10 degrees, 20 degrees, 30 degrees, 40 degrees, 50 degrees, 60 degrees, 70 degrees, 80 degrees, or any value between any two of these values. It is worth noting that the acute angle at the intersection is not limited to the aforementioned numerical range as long as it can fix the position of the control handle 240. In some examples, the intersection angle θ of the second locking slot 233 and the main groove 231 is defined by the direction of second locking slot 233 perpendicular to axis AX; meanwhile, the second locking slot 233 has a certain length, so that when cleaning the lens of the imaging element, the control handle 240 can reciprocate a certain distance in the second locking slot 233, and the tube 210 and the cleaning element 100 are driven to rotate by a certain distance along the direction of the axis AX to clean the lens. That is, the distance that the control handle 240 reciprocates in the second locking slot 233 and the distance that the cleaning element 100 is rotated along the direction of the axis AX correspond to the length of the second locking slot 233.

Please refer to Fig. 4, Fig. 4 depicts a perspective view showing the use of the guiding element of the imaging element cleaning device according to another embodiment of the present disclosure. The difference between Fig. 1 and Fig. 4 is that the guiding groove 230 is linear, that is, the guiding groove 230 extends along the direction of the axis AX, so that the imaging element passes through the cleaning element 100 for clean. Specifically, the main groove 231 is linear as above mentioned. The first locking slot 232 is disposed at the proximal end 2312 of the main groove 231, when not cleaning (the imaging element is in use), the position of the control handle 240 is fixed, and the cleaning element 100 is also fixed at the same time. The second locking slot 233 is disposed at the distal end 2311 of the main groove 231, when cleaning (the imaging element is not in use), the area where the control handle 240 can be moved is limited; such as, after the control handle 240 slides along the main groove 231 to the second locking slot 233 and slides back and forth in the second locking slot 233, at this time, the cleaning element 100 is also rotated in *situ* to clean the lens LEN of the imaging element IE (as shown in FIG. 8 ); or the control handle 240 may be temporarily stopped during cleaning to fix the position of the control handle 240. In some examples, the intersection angle θ of the first locking slot 232 and the main groove 231 and the intersection angle θ of the second locking slot 233 and the main groove 231 are right angles, such as 90 degrees. In some examples, the second locking slot 233 extends perpendicularly to both sides of the main groove 231 at the intersection and has a certain length, so that when cleaning the lens of the imaging element, the control handle 240 can reciprocate a certain distance in the second locking slot 233, and the tube 210 and the cleaning element 100 are driven to rotate by a certain distance along the direction of the axis AX to clean the lens. That is, the distance that the control handle 240 reciprocates in the second locking slot 233 and the distance that the cleaning element 100 is rotated along the direction of the axis AX correspond to the length of the second locking slot 233.

The control handle 240 protrudes from the proximal end 212 of the tube 210 and correspondingly passes through the guiding groove 230, the control handle 240 is moved along the guiding groove 230 to drive the tube 210 and the cleaning element 100 to clean. In some examples, the control handle 240 is moved along the main groove 231. When the control handle 240 is moved to either of two opposite ends of the first locking slot 232, the position of the control handle 240 is fixed. In some examples, the shape of the first locking slot 232 is for the control handle 240 to pass through and fix, and is not limited to that disclosed in the drawings of this disclosure. When the control handle 240 is moved to the second locking slot 233, since the second locking slot 233 has a certain length, the control handle 240 can reciprocate a certain distance in the second locking slot 233 when cleaning the lens of the imaging element, and the tube 210 and the cleaning element 100 are driven to rotate by a certain distance along the direction of the axis AX to clean the lens.

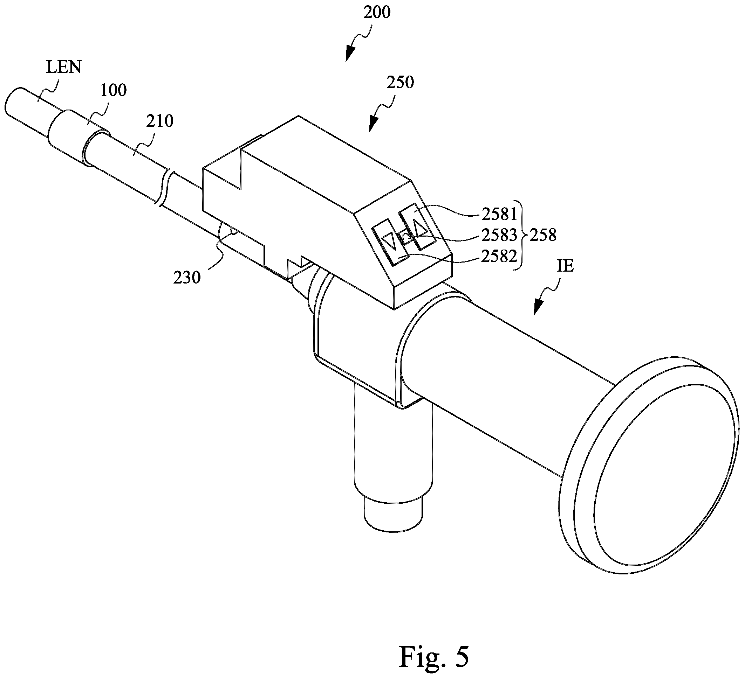

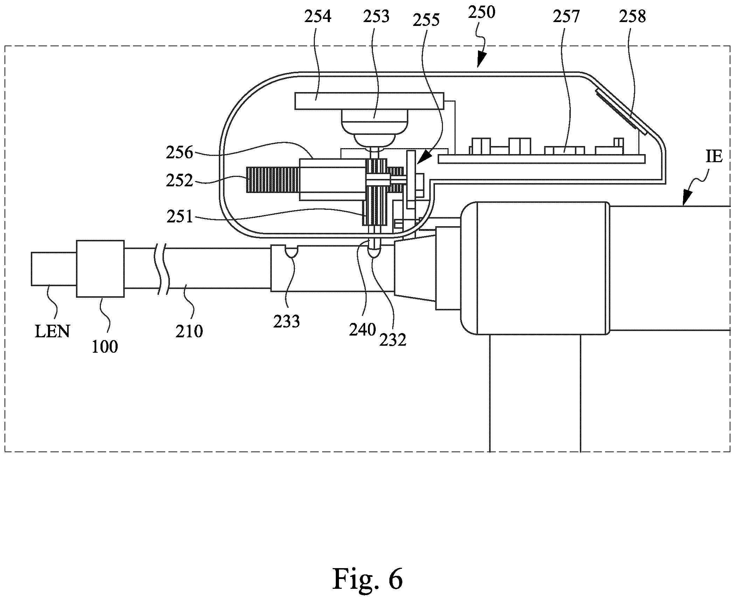

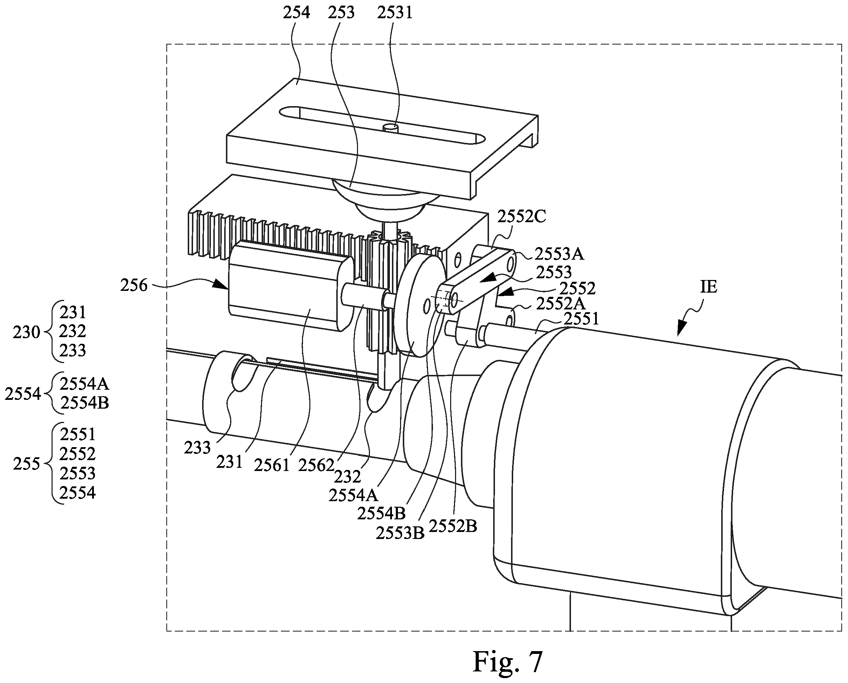

Please refer to Figs. 5 to 7, Fig. 5 depicts a perspective view of the imaging element cleaning device with an automatic control driving device according to an embodiment of the present disclosure, Fig. 6 depicts a side view and a partial cross-sectional view of Fig. 5, and Fig. 7 depicts a partial cross-sectional view of Fig. 5. The driving component 200 further comprises an automatic control driving device 250, the automatic control driving device 250 is operably connected to the control handle 240, to drive the control handle 240 moving along the guiding groove 230 to drive the tube 210 and the cleaning element 100 to move for cleaning. In some examples, the automatic control driving device 250 includes a gear shaft 251, a rack 252, a first motor 253, a single axis slide rail 254, a cam group 255, a second motor 256, a processor 257, and a button group 258. One end of the gear shaft 251 is sleeved on the control handle 240, the other one end is pivotally connected the first motor 253. An outer wall of the gear shaft 251 has a plurality of teeth that mesh with the rack 252. The single axis slide rail 254 is disposed over the first motor 253, an end of the first motor 253 away from the gear shaft 251 has a protruding rod 2531, the protruding rod 2531 is disposed in the single axis slide rail 254 to limit a sliding position of the first motor 253, such as the control handle 240 is moved along the main groove 231 of the guiding groove 230.

The cam group 255 includes a fixed axis 2551, a first connecting rod 2552, a second connecting rod 2553, and a disc 2554. A proximal end of the fixed axis 2551 is connected to the imaging element IE, a distal end of the fixed axis 2551 is connected to the first connecting rod 2552. In some examples, first connecting rod 2552 is L-shaped connecting rod, and it sequentially includes a free end 2552A, a turning end 2552B, and a connecting end 2552C. The distal end of the fixed axis 2551 is connected to the turning end 2552B of the first connecting rod 2552. The connecting end 2552C of the first connecting rod 2552 is connected to a proximal end of the rack 252. In some examples, second connecting rod 2553 is a straight connecting rod including a first end 2553A and a second end 2553B corresponding to each other. The first end 2553A of the second connecting rod 2553 is connected to the connecting end 2552C of the first connecting rod 2552. The disc 2554 includes a body 2554A and a convex 2554B protrudes from the body 2554A adjacent to a surface of the second connecting rod 2553, and the second end 2553B of the second connecting rod 2553 is connected to the convex 2554B. The second motor 256 includes a motor body 2561 and a transmission shaft 2562 passing through the motor body 2561, and an end of the transmission shaft 2562 away from the motor body 2561 passes through a center of the body 2554A of the disc 2554. The automatic control driving device 250 is driven by the cam group 255 and the second motor 256 to move the control handle 240 within the first locking slot 232 or the second locking slot 233.

The processor 257 is electrically connected to the first motor 253, the second motor 256, and the button group 258. In some examples, the button group 258 includes a distal end button 2581, a proximal end button 2582, and a left-right swing button 2583 disposed between the distal end button 2581 and the proximal end button 2582. The distal end button 2581, the proximal end button 2582, and the left-right swing button 2583 are electrically connected to the processor 257, respectively. In addition, the automatic control driving device 250 in the present disclosure is implemented as shown in Figs. 5 to 7. In other examples, the purpose of the automatic control driving device 250 is to drive the control handle 240. Other types of the automatic control driving devices that can achieve this purpose may also be used, and are not limited to those disclosed in the drawings of the present disclosure.

In order to describe the method of operating the imaging element cleaning device in detail, the following will be illustrated with Figs. 6 to 9.

Please continue to refer to Fig. 8, Fig. 8 depicts a perspective view showing the use of the guiding element of the imaging element cleaning device according to an embodiment of the present disclosure. A method of operating the imaging element cleaning device 10 includes providing the imaging element cleaning device 10 as above mentioned and the imaging element IE passing through the tube 210 of the driving component 200 and the slit part 130 of the cleaning element100.

Specifically, first, the imaging element IE (e.g., an endoscope sequentially having a lens, an insertion tube, and a handling portion) is passed through the tube 210 of the driving component 200 and the slit part 130 of the cleaning element 100, so that the lens LEN of the imaging element IE protrudes from the cleaning element 100 for image detection. Meanwhile, the control handle 240 is located in the first locking slot 232 and positioned at one end of the first locking slot 232 to fix the imaging element cleaning device 10.

Next, when the lens LEN encounters contaminants or tissue fluid adhesion causing the image presented by the imaging element IE to be blurred, the control handle 240 is moved out from the first locking slot 232 to the main groove 231 and rotated toward the second locking slot 233. Meanwhile, the tube 210 and the cleaning element 100 are driven to rotate and move toward the lens LEN so that the lens LEN passes through the slit part 130.

Next, when the control handle 240 is moved to the second locking slot 233, the lens LEN abuts against the protrusion 123 of the cleaning part 120 (as shown in Fig. 2A). Next, the control handle 240 reciprocates in the second locking slot 233, and the tube 210 and the cleaning element 100 are driven to rotate around the axis AX of the flexible sleeve 110. Meanwhile, the control handle 240 is driven to move and rotate the tube 210 and the protrusion 123 of the cleaning element 100 to clean the lens LEN, so that the contaminants or tissue fluid flow out along the slit 131 of the slit part 130.

In some examples as shown in Fig. 4, the difference from Fig. 8 is that the main groove 231 is linear. Specifically, when the lens LEN encounters contaminants or tissue fluid adhesions that blur the image presented by the imaging element IE, the control handle 240 is moved out from the first locking slot 232 to the main groove 231 and moved parallel to the second locking slot 233. Next, since the second locking slot 233 extends perpendicularly to both sides of the main groove 231 at the intersection, the control handle 240 reciprocates in the second locking slot 233, driving the tube 210 and the cleaning element 100 to rotate around the axis AX of the flexible sleeve 110 to clean the lens.

In some examples, please refer back to Figs. 5, 6, 7, and 9, Fig. 9 depicts a side view showing the use of the automatic control driving device according to another embodiment of the present disclosure. The difference from Fig. 8 is that the driving component 200 further comprises the automatic control driving device 250. When the imaging element cleaning device 10 having the automatic control driving device 250 is used, the processor 257 receives an electrical signal from the distal end button 2581 of the button group 258, and the processor 257 transmits the signal and the first motor 253 is driven by the processor 257. Next, the gear shaft 251 is simultaneously driven by the first motor 253, and the control handle 240 is driven to move in the main groove 231 toward the second locking slot 233 along the single axis slide rail 254 and the rack 252, thereby driving the tube 210 to move and moving the cleaning element 100 toward the direction of the lens LEN of the imaging element IE.

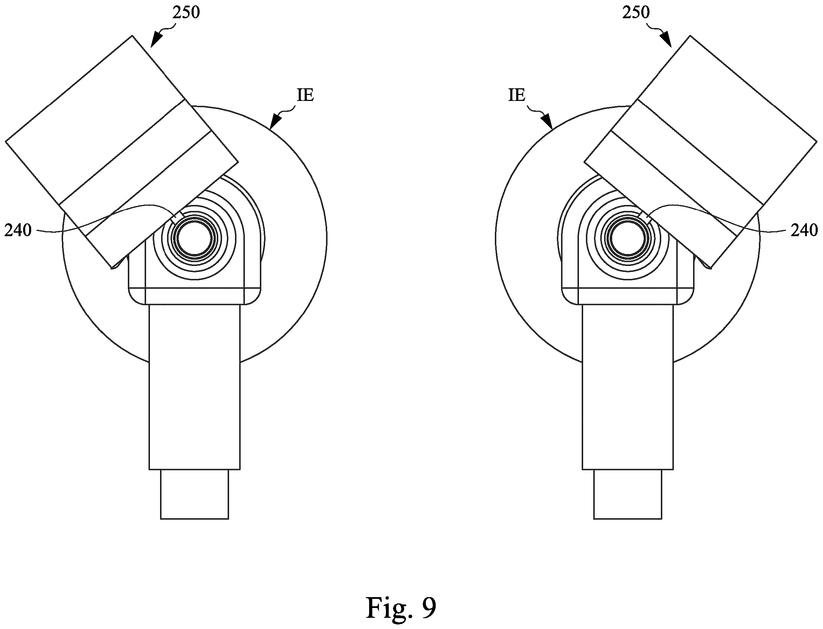

When processor 257 receives a moving signal from the proximal end button 2582 of the button group 258, the above-mentioned action is the other way around. When processor 257 receives the electrical signal from the left-right swing button 2583 of the button group 258, the processor 257 transmits the signal and the second motor 256 is driven by the processor 257. Next, the cam group 255 is simultaneously driven by the second motor 256, and the control handle 240 is driven to reciprocate in the second locking slot 233, so that when looking from the distal end of the tube 210 toward the proximal end (as shown in FIG. 9), the automatic control driving device 250 is in a left swing or right swing state. Meanwhile, the cleaning element 100 is driven to rotate along the direction of the axis AX to clean the lens LEN of the imaging element IE, achieving labor saving and automatic control.

Accordingly, the present disclosure provides the imaging element cleaning device and a method of operating the same, which can clean the lens and shorten the cleaning time by using the cleaning element and the driving component.

## Claims

1. An imaging element cleaning device (10) for cleaning a lens (LEN) of an imaging element (IE), the imaging element cleaning device (10) comprising:
a cleaning element (100) comprising:
a flexible sleeve (110) being hollow, and comprising a distal end (111) and a proximal end (113) opposite to the distal end (111);
a cleaning part (120) partially covering the distal end (111) of the flexible sleeve (110); and
a slit part (130) penetrating through the cleaning part (120); and
a driving component (200) comprising:
a tube (210) comprising a distal end (211) and a proximal end (212) opposite to the distal end (211) for the imaging element (IE) to pass through, and the flexible sleeve (110) of the cleaning element (110) sleeved on the distal end (211) of the tube (210), wherein when the driving component (200) is driven to move the cleaning element (100), the lens (LEN) of the imaging element (IE) passes through the slit part (130) for cleaning by the cleaning part (120).

2. The imaging element cleaning device (10) of claim 1, wherein a material of the cleaning element (100) comprises silicone, rubber, resin, or a combination thereof.

3. The imaging element cleaning device (10) of claims 1 or 2, wherein the flexible sleeve (110) is cylindrical and has an axis (AX), and the cleaning part (120) includes:
an edge (121) located at a distal end of the cleaning part (120);
at least one slice (122) extending from an edge (112) of the flexible sleeve (110) at the distal end (111) toward the axis (AX) of the flexible sleeve (110), wherein the at least one slice (122) has an outer surface (1221) and an inner surface (1222) relative to the outer surface (1221); and
at least one protrusion (123) disposed on the inner surface (1222) of the at least one slice (122).

4. The imaging element cleaning device (10) of claims 1 or 2, wherein the flexible sleeve (110) is cylindrical and has an axis (AX), and the cleaning part (120) includes:
an edge (121) located at a distal end of the cleaning part (120);
at least four slices (122) extending from an edge (112) of the flexible sleeve (110) at the distal end (111) toward the axis (AX) of the flexible sleeve (120), wherein each one of the at least four slices (122) has an outer surface (1221) and an inner surface (1222) relative to the outer surface (1221); and
at least one protrusion (123) disposed on the inner surface (1222) of one of the at least four slices (122).

5. The imaging element cleaning device (10) of claim 4, wherein a number of the at least one protrusion (123) is at least four protrusions (123) respectively disposed on the inner surfaces (1222) of the at least four slices (122).

6. The imaging element cleaning device (10) of claim 5, wherein each one of the at least four protrusions (123) comprises a plurality of bumps (1231).

7. The imaging element cleaning device (10) of claim 6, wherein a distribution pattern of the plurality of bumps (1231) comprises evenly distributed on the inner surface (1222) of each slice (122), concentratedly distributed on the inner surface (1222) of each slice (122) adjacent to the axis (AX) of the flexible sleeve (110), or concentratedly distributed on the inner surface (1222) of each slice (122) and away from the axis (AX) of the flexible sleeve (110).

8. The imaging element cleaning device (10) of claim 5, wherein each one of the at least four protrusions (123) comprises a bump (1231), the bump (1231) has a height (H) increasing from the inner surface (1222) of each of the at least four slices (122) adjacent to the edge (121) of the cleaning part (120) toward a direction of the axis (AX).

9. The imaging element cleaning device (10) of any one of claims 4 to 8, wherein the slit part (130) comprises at least two slits (131) intersecting each other, and separating the at least four slices (122).

10. The imaging element cleaning device (10) of claim 9, wherein the at least two slits (131) intersect each other symmetrically or asymmetrically.

11. The imaging element cleaning device (10) of any one of claims 1 to 10, wherein the driving component (200) further comprises:
a guiding element (220) sleeved at the proximal end (212) of the tube (210);
a guiding groove (230) penetrating through the guiding element (220); and
a control handle (240) protruding from the proximal end (212) of the tube (210) and correspondingly passing through the guiding groove (230), the control handle (240) moving along the guiding groove (230) to drive the tube (210) and the cleaning element (100) to move for cleaning.

12. The imaging element cleaning device (10) of claim 11, wherein the guiding groove (230) is linear or spiral.

13. The imaging element cleaning device (10) of claims 11 or 12, wherein the guiding groove (230) comprises:
a main groove (231), the control handle (240) moved along the main groove (231);
a first locking slot (232) disposed at a proximal end (2312) of the main groove (231) to fix the control handle (240); and
a second locking slot (233) disposed at a distal end (2311) of the main groove (231) and comprising a bottom portion (2331), the bottom portion (2331) being away from the distal end (2311) of the main groove (231), wherein
when cleaning the lens (LEN) of the imaging element (IE), the control handle (240) slides back and forth in the second locking slot (233) to rotate the cleaning element (100); or
when the cleaning is temporarily stopped, the control handle (240) is fixed to the bottom portion (2331) of the second locking slot (233).

14. The imaging element cleaning device (10) of any one of claims 11 to 13, wherein the driving component (200) further comprises an automatic control driving device (250) operably connected to the control handle (240) to drive the control handle (240) moving along the guiding groove (230) to drive the tube (210) and the cleaning element (100) to move for cleaning.

15. A method of operating imaging element cleaning device (10), comprising:
providing the imaging element cleaning device (10) as claimed in any one of claims 1 to 14 and an imaging element (IE) passing through the tube (210) of the driving component (200) and the slit part (130) of the cleaning element (100),
wherein when a lens (LEN) of the imaging element (IE) is contaminated, the driving component (200) is driven to move the cleaning element (100), and the lens (LEN) of the imaging element (IE) is moved toward a proximal end (212) of the tube (210) and passes through the slit part (130) to be cleaned by the cleaning part (120).

## Amended claims

### Amended claims in accordance with Rule 137(2) EPC.

1. An imaging element cleaning device (10) for cleaning a lens (LEN) of an imaging element (IE), the imaging element cleaning device (10) comprising:
a cleaning element (100) comprising:
a flexible sleeve (110) being hollow, and comprising a distal end (111) and a proximal end (113) opposite to the distal end (111);
a cleaning part (120) partially covering the distal end (111) of the flexible sleeve (110); and
a slit part (130) penetrating through the cleaning part (120); and
a driving component (200) comprising:
a tube (210) comprising a distal end (211) and a proximal end (212) opposite to the distal end (211) for the imaging element (IE) to pass through, and the flexible sleeve (110) of the cleaning element (100) sleeved on the distal end (211) of the tube (210), wherein when the driving component (200) is driven to move the cleaning element (100), the lens (LEN) of the imaging element (IE) passes through the slit part (130) for cleaning by the cleaning part (120).

2. The imaging element cleaning device (10) of claim 1, wherein a material of the cleaning element (100) comprises silicone, rubber, resin, or a combination thereof.

3. The imaging element cleaning device (10) of claims 1 or 2, wherein the flexible sleeve (110) is cylindrical and has an axis (AX), and the cleaning part (120) includes:
an edge (121) located at a distal end of the cleaning part (120);
at least one slice (122) extending from an edge (112) of the flexible sleeve (110) at the distal end (111) toward the axis (AX) of the flexible sleeve (110), wherein the at least one slice (122) has an outer surface (1221) and an inner surface (1222) relative to the outer surface (1221); and
at least one protrusion (123) disposed on the inner surface (1222) of the at least one slice (122).

4. The imaging element cleaning device (10) of claims 1 or 2, wherein the flexible sleeve (110) is cylindrical and has an axis (AX), and the cleaning part (120) includes:
an edge (121) located at a distal end of the cleaning part (120);
at least four slices (122) extending from an edge (112) of the flexible sleeve (110) at the distal end (111) toward the axis (AX) of the flexible sleeve (110), wherein each one of the at least four slices (122) has an outer surface (1221) and an inner surface (1222) relative to the outer surface (1221); and
at least one protrusion (123) disposed on the inner surface (1222) of one of the at least four slices (122).

5. The imaging element cleaning device (10) of claim 4, wherein a number of the at least one protrusion (123) is at least four protrusions (123) respectively disposed on the inner surfaces (1222) of the at least four slices (122).

6. The imaging element cleaning device (10) of claim 5, wherein each one of the at least four protrusions (123) comprises a plurality of bumps (1231).

7. The imaging element cleaning device (10) of claim 6, wherein a distribution pattern of the plurality of bumps (1231) comprises evenly distributed on the inner surface (1222) of each slice (122), concentratedly distributed on the inner surface (1222) of each slice (122) adjacent to the axis (AX) of the flexible sleeve (110), or concentratedly distributed on the inner surface (1222) of each slice (122) and away from the axis (AX) of the flexible sleeve (110).

8. The imaging element cleaning device (10) of claim 5, wherein each one of the at least four protrusions (123) comprises a bump (1231), the bump (1231) has a height (H) increasing from the inner surface (1222) of each of the at least four slices (122) adjacent to the edge (121) of the cleaning part (120) toward a direction of the axis (AX).

9. The imaging element cleaning device (10) of any one of claims 4 to 8, wherein the slit part (130) comprises at least two slits (131) intersecting each other, and separating the at least four slices (122).

10. The imaging element cleaning device (10) of claim 9, wherein the at least two slits (131) intersect each other symmetrically or asymmetrically.

11. The imaging element cleaning device (10) of any one of claims 1 to 10, wherein the driving component (200) further comprises:
a guiding element (220) sleeved at the proximal end (212) of the tube (210);
a guiding groove (230) penetrating through the guiding element (220); and
a control handle (240) protruding from the proximal end (212) of the tube (210) and correspondingly passing through the guiding groove (230), the control handle (240) moving along the guiding groove (230) to drive the tube (210) and the cleaning element (100) to move for cleaning.

12. The imaging element cleaning device (10) of claim 11, wherein the guiding groove (230) is linear or spiral.

13. The imaging element cleaning device (10) of claims 11 or 12, wherein the guiding groove (230) comprises:
a main groove (231), the control handle (240) moved along the main groove (231);
a first locking slot (232) disposed at a proximal end (2312) of the main groove (231) to fix the control handle (240); and
a second locking slot (233) disposed at a distal end (2311) of the main groove (231) and comprising a bottom portion (2331), the bottom portion (2331) being away from the distal end (2311) of the main groove (231), wherein
when cleaning the lens (LEN) of the imaging element (IE), the control handle (240) slides back and forth in the second locking slot (233) to rotate the cleaning element (100); or
when the cleaning is temporarily stopped, the control handle (240) is fixed to the bottom portion (2331) of the second locking slot (233).

14. The imaging element cleaning device (10) of any one of claims 11 to 13, wherein the driving component (200) further comprises an automatic control driving device (250) operably connected to the control handle (240) to drive the control handle (240) moving along the guiding groove (230) to drive the tube (210) and the cleaning element (100) to move for cleaning.

15. A method of operating imaging element cleaning device (10), comprising:
providing the imaging element cleaning device (10) as claimed in any one of claims 1 to 14 and an imaging element (IE) passing through the tube (210) of the driving component (200) and the slit part (130) of the cleaning element (100),
wherein when a lens (LEN) of the imaging element (IE) is contaminated, the driving component (200) is driven to move the cleaning element (100), and the lens (LEN) of the imaging element (IE) is moved toward a proximal end (212) of the tube (210) and passes through the slit part (130) to be cleaned by the cleaning part (120).
